# Europäisches Patentamt

# European Patent Office

# Office européen des brevets

(19)

(11) Numéro de publication: **0 012 640
B1**

(12)

# FASCICULE DE BREVET EUROPEEN

(45) Date de publication du fascicule de brevet:
**05.05.82**

(51) Int. Cl.³: **C 07 J 9/00**

(21) Numéro de dépôt: **79400872.2**

(22) Date de dépôt: **14.11.79**

(54) **Procédé de purification de l'acide chénodésoxycholique et produits obtenus par ce procédé.**

(30) Priorité: **11.12.78 FR 7834767**

(43) Date de publication de la demande:
**25.06.80 Bulletin 80/13**

(45) Mention de la délivrance du brevet:
**05.05.82 Bulletin 82/18**

(84) Etats contractants désignés:
**CH DE GB IT NL**

(56) Documents cités:
**FR-A-2 346 370**

**PATENT ABSTRACTS OF JAPAN,
vol. 3, n° 14, 8 février 1979,
page 48C36.**

(73) Titulaire: **ROUSSEL-UCLAF, 102, route de Noisy Boîte
postale no.9, F-93230 Romainville (FR)**

(72) Inventeur: **Pavan, Charles, 26-28 Grande Rue Charles de
Gaulle, F-94130 Nogent sur Marne (FR)**
Inventeur: **Bulidon, Jacques, Place du Monument aux
Morts, F-86350 Usson du Poitou (FR)**

(74) Mandataire: **Douetteau, Pierre et ai, Boîte postale
no 9 102, route de Noisy, F-93230-Romainville (FR)**

## Procédé de purification de l'acide chénodésoxycholique et produits obtenus par ce procédé

La présente invention concerne un nouveau procédé de purification de l'acide chénodésoxycholique brut, ainsi que les produits obtenus par ce procédé.

L'acide chénodésoxycholique ou acide dihydroxy -3α, 7α (5β) cholanique, permet la dissolution des calculs biliaires cholestéroliques et est utilisé en médecine humaine dans le traitement de la lithiase biliaire.

L'acide chénodésoxycholique est généralement préparé à partir d'une substance de départ naturelle. Il est couramment préparé à partir d'acide cholique, ou acide trihydroxy -3α, 7α, 12α (5β) cholanique (extrait lui-même de bile animale), dont la fonction hydroxy en position 12 est éliminée par une suite de réactions [Feiser et coll., J.A.C.S. 72: 5530 (1950)].

L'acide chénodésoxycholique brut renferme, du fait de sa préparation, des produits secondaires, notamment d'autres acides biliaires tels que l'acide lithocholique. La présence de ces impuretés dans un produit administré en thérapeutique prolongée, comme c'est le cas pour l'acide chénodésoxycholique, produirait des effets secondaires indésirables.

Il importe donc d'administrer un produit de très haute pureté, et pour cela, de disposer d'un procédé de purification de l'acide chénodésoxycholique qui permette d'obtenir un produit très pur.

La demande de brevet japonais JP-A-5 313 794.5 propose de purifier l'acide chénodésoxycholique en le traitant par un mélange d'ester d'alkyl inférieur de l'acide acétique et d'alcane. L'ester d'alkyl inférieur de l'acide acétique peut être par exemple l'acétate de méthyle ou l'acétate d'éthyle, et l'alcane peut être par exemple l'hexane, l'heptane, l'octane ou le pentane. Le mélange de solvant est saturé d'eau et les solvants sont par exemple l'acétate d'éthyle et le n-hexane (2 : 1).

Il est proposé par la demande allemande OLS 2 302 744 de purifier l'acide chénodésoxycholique en traitant le produit brut en solution méthanolique, par un sel de calcium, ou de strontium, en alcalinisant, puis en traitant par un acide le sel de calcium ou de strontium formé et en extrayant l'acide chénodésoxycholique.

Il est proposé également, par le brevet français n° 2 273 011, pour purifier l'acide chénodésoxycholique, de soumettre à une extraction continue liquide - liquide l'acide brut sous la forme de son sel alcalin aqueux, de préférence son sel de sodium ou de potassium, par un solvant organique, de préférence par l'acétate d'éthyle ou par un mélange d'isobutanol et de toluène, d'acidifier ensuite la solution saline par un acide dilué, de préférence l'acide chlorhydrique, d'extraire et de précipiter l'acide chénodésoxycholique par de l'eau.

La demande de brevet français 2 269 957 propose pour purifier l'acide chénodésoxycholique, de préparer un complexe d'inclusion de l'acide brut et d'un clathrate de solvant organique tel que notamment l'acétate d'éthyle ou l'heptane, puis d'éliminer ensuite le solvant organique.

Le brevet français n° 2 346 370, décrit la purification de l'acide chénodésoxycholique brut par cristallisation dans l'acétonitrile.

Le but de la présente invention est de fournir un procédé industriel de purification, de l'acide chénodésoxycholique, qui permette d'obtenir un produit qui réponde aux exigences actuelles en matière de pureté pour un médicament, et évite les manifestations d'intolérance et les effets secondaires dus à la présence en quantité trop importante, même s'il s'agit de très faibles pourcentages, d'impuretés gênantes tel que l'acide lithocholique.

Cette exigence de pureté s'impose particulièrement dans le cas de l'acide chénodésoxycholique, qui est un médicament administré au patient quotidiennement pendant de nombreux mois.

C'est ainsi, par exemple, que le procédé de l'invention permet à partir d'un produit brut contenant environ 2% d'impuretés essentiellement constituées d'acides biliaires autres que l'acide chénodésoxycholique, dont environ 0,5% d'acide 3α-hydroxy cholanique ou acide lithocholique, d'obtenir un produit purifié qui renferme moins de 1% d'acides biliaires étrangers dont moins de 0,1% d'acide lithocholique.

Un tel degré de pureté ne pouvait être atteint, dans des conditions de rendement satisfaisantes sur le plan industriel, avec les procédés de purification antérieurement connus.

La présente demande a ainsi pour objet un procédé de purification de l'acide dihydro-3α, 7α (5β) cholanique, caractérisé en ce que l'on fait cristalliser l'acide dihydroxy-3α, 7α (5β) cholanique à purifier dans le chlorure de méthylène, recueille le produit cristallisé formé et recupère de celui-ci l'acide -3α, 7α dihydroxy (5β) cholanique purifié.

Selon l'opinion de la demanderesse, le produit cristallisé formé est vraisemblablement constitué d'acide dihydroxy-3α, 7α (5β) cholanique pur sous une forme cristalline particulière, ou d'un solvant de cet acide. Mais bien entendu, il s'agit là d'une hypothèse qui ne saurait en aucune façon limiter la portée de l'invention.

La cristallisation dans le chlorure de méthylène permet d'obtenir des rendements de purification meilleurs que ceux que l'on pourrait obtenir avec d'autres solvants.

La présente demande a plus particulièrement pour objet un procédé tel que défini ci-dessus, caractérisé en ce que la cristallisation est réalisée dans un système de cristallisation constitué par le chlorure de méthylène et un alcool.

Dans des conditions préférentielles de mise en oeuvre du procédé de l'invention, on opère comme suit.

- Le système de cristallisation est constitué par le chlorure de méthylène et un alcool aliphatique.
- L'alcool du système de cristallisation peut être notamment le méthanol, l'éthanol, le propanol, l'isopropanol, le butanol, un glycol, un alcool substitué tel que l'éthoxy-éthanol.
- On dissout d'abord l'acide dihydroxy-$3\alpha$, $7\alpha$ ($5\beta$) cholanique brut dans un mélange éthanol-chlorure de méthylène, puis ajoute ensuite du chlorure de méthylène.
- Le système de cristallisation est utilisé dans les rapports de 0,1 : 1 à 0,7 : 1 d'éthanol et 5,9 : 1 à 7,2 : 1 de chlorure de méthylène, par rapport à l'acide dihydroxy-$3\alpha$, $7\alpha$ ($5\beta$) cholanique.
- La cristallisation est réalisée à chaud notamment à la température de reflux du milieu réactionnel.
- On récupère l'acide dihydroxy-$3\alpha$, $7\alpha$ ($5\beta$) cholanique par séchage du produit cristallisé. Par «séchage», on entend par action de la chaleur, du vide ou par ventilation.
- On peut également récupérer l'acide dihydroxy-$3\alpha$, $7\alpha$ ($5\beta$) cholanique du produit cristallisé par recristallisation dans un autre solvant ou système solvant, ou par dissolution dans un solvant ou système solvant, notamment un mélange acétone-eau, évaporation partielle du solvant ou système solvant et isolement du produit cristallisé obtenu.

La présente invention a également pour objet l'acide dihydroxy-$3\alpha$, $7\alpha$ ($5\beta$) cholanique purifié, caractérisé en ce qu'il a été préparé selon le procédé défini ci-dessus.

La présente invention a également pour objet l'acide dihydroxy-$3\alpha$, $7\alpha$ ($5\beta$) cholanique purifié, caractérisé en ce qu'il renferme moins de 1% d'impuretés, et notamment l'acide dihydroxy-3a, $7\alpha$ ($5\beta$) cholanique purifié, caractérisé en ce qu'il renferme moins de 0,1% d'acide $3\alpha$-hydroxy cholanique.

La présente invention a enfin pour objet l'acide dihydroxy-$3\alpha$, $7\alpha$ ($5\beta$) cholanique sous la forme du produit cristallisé formé dans le chlorure de méthylène, selon le procédé défini ci-dessus.

L'exemple donné ci-après illustre l'invention sans toutefois la limiter.

Exemple

On dissout sous vive agitation 100 g d'acide chénodésoxycholanique brut contenant environ 2% d'impuretés essentiellement constituées d' acides biliaires étrangers dont environ 5‰ d'acide lithocholique, dans un mélange de 40 cm3 d'éthanol 100° dénaturé au butanol, 210 cm3 de chlorure de méthylène et 0,1 g d'acétate de sodium à la température de 20 - 25°C, porte au reflux pendant 20 minutes, ajoute sous vive agitation en 10 minutes environ tout en maintenant au reflux, 450 cm3 de chlorure de méthylène ce qui entraîne une cristallisation abondante, maintient au reflux pendant une heure, refroidit la suspension en trois heures jusqu'à une température comprise entre 0°C et −2°C, agite pendant 2 heures à cette température, essore et lave trois fois avec un mélange à 0°C/−2°C de 2,8 cm3 d'éthanol 100° dénaturé au butanol, et 47,2 cm3 de chlorure de méthylène. On sèche en étuve ventilée à 40°C et obtient 79 g d'acide chénodésoxycholique purifié qui fond à 123°C et dont la teneur en chlorure de méthylène est inférieure à 0,1%.

On redissout le produit obtenu dans 316 cm3 d'acétone à 5% d'eau; on agite à 20 - 25°C jusqu'à dissolution, ajoute 0,79 g de charbon actif, agite pendant 15 minutes, filtre et rince avec 79 cm3 d'acétone à 5% d'eau. On ajoute au filtrat 74,2 cm3 d'eau, porte à ébullition, puis distille à pression ordinaire 237 cm3 de mélange eau-acétone, ajoute 237 cm3 d'eau, laisse refroidir à 38°C, agite pendant 2 heures, puis laisse refroidir à 20 - 22°C, agite pendant 2 heures, essore, lave, avec trois fois 79 cm3 d'eau, sèche et obtient 76,6 g d'acide chénodésoxycholique purifié. (On conserve les eaux de lavage et les eaux mères de cristallisation, en vue de l'isolement d'un deuxième jet.)

$[\alpha]_D = +10° 5 \pm 1°$ (C = 10%, dioxane).

Par chromatographie en couche mince, on voit que le produit purifié obtenu renferme moins de 1% d'acides biliaires autres que l'acide chénodésoxycholique (notamment acide cholique, acide $3\alpha$, $7\alpha$-dihydroxy-12-cétocholanique, acide $3\alpha$-hydroxycholanique ou acide lithocholique, et $3\alpha$, $12\alpha$-dihydroxycholanique ou acide désoxycholique) et notamment moins de 1‰ d'acide lithocholique.

[La chromatographie en couche mince a été réalisée de la façon suivante: on utilise des plaques de silice. Lacide chénodésoxycholique est dissous dans l'acétone (4%). On effectue un dépôt de 10 μL. On utilise comme solvant de migration le système solvant:
- isooctane - acétate d'éthyle - acide acétique pur (5-2-2) - On laisse migrer en cuve saturée sur 15 centimètres et laisse migrer ensuite pendant une heure. On révèle par pulvérisation d' acide sulfurique à 10% dans l'éthanol, chauffe à 100°C et examine à la lumière U.V.].

## Revendications

1. Procédé de purification de l'acide dihydroxy-$3\alpha$, $7\alpha$, ($5\beta$) cholanique, caractérisé en ce que l'on fait cristalliser l'acide dihydroxy-$3\alpha$, $7\alpha$ ($5\beta$) cholanique à purifier, dans le chlorure de méthylène, recueille le produit cristallisé formé, et récupère de celui-ci l'acide dihydroxy-$3\alpha$, $7\alpha$ ($5\beta$)-cholanique purifié.

2. Procédé selon la revendication 1, caractérisé en ce que la cristallisation est réalisée dans un système de cristallisation constitué par le chlorure de méthylène et un alcool.

3. Procédé selon la revendication 1 ou 2, caractérisé en ce que le système de cristallisation est constitué par le chlorure de méthylène et un alcool aliphatique.

4. Procédé selon la revendication 3, caractérisé en ce que l'alcool aliphatique est l'éthanol.

5. Procédé selon la revendication 4, caractérisé en ce que le système de cristallisation est utilisé dans les rapports de 0,1 : 1 à 0,7 : 1 d'éthanol et 5,9 : 1 à 7,2 : 1 de chlorure de méthylène, par rapport à l'acide dihydroxy-3$\alpha$, 7$\alpha$ (5$\beta$) cholanique.

6. Procédé selon l'une quelconque des revendications 1 à 5, caractérisé en ce que la cristallisation est réalisée à chaud.

7. Procédé selon la revendication 1 ou 2, caractérisé en ce que l'on récupère l'acide dihydroxy-3$\alpha$, 7$\alpha$ (5$\beta$) cholanique par séchage du produit cristallisé.

8. Procédé selon la revendication 1 ou 2, caractérisé en ce que l'on récupère l'acide dihydroxy-3$\alpha$, 7$\alpha$ (5$\beta$) cholanique par séchage du produit cristallisé, puis dissolution du produit obtenu dans un mélange acétone-eau, évaporation partielle dudit mélange, puis isolement du produit cristallisé obtenu.

## Patentansprüche

1. Verfahren zur Reinigung von Dihydroxy-3$\alpha$,-7$\alpha$,5$\beta$-cholansäure, dadurch gekennzeichnet, dass man die zu reinigende Dihydroxy-3$\alpha$,7$\alpha$,-5$\beta$-cholansäure in Methylenchlorid kristallisiert, das gebildete kristallisierte Produkt sammelt und aus diesem die gereinigte Dihydroxy-3$\alpha$,7$\alpha$,-5$\beta$-cholansäure gewinnt.

2. Verfahren gemäss Anspruch 1, dadurch gekennzeichnet, dass die Kristallisation in einem Kristallisationssystem durchgeführt wird, das aus Methylenchlorid und einem Alkohol besteht.

3. Verfahren gemäss Anspruch 1 oder 2, dadurch gekennzeichnet, dass das Kristallisationssystem aus Methylenchlorid und einem aliphatischen Alkohol besteht.

4. Verfahren gemäss Anspruch 3, dadurch gekennzeichnet, dass der aliphatische Alkohol Äthanol ist.

5. Verfahren gemäss Anspruch 4, dadurch gekennzeichnet, dass das Kristallisationssystem in Verhältnissen von 0,1 : 1 bis 0,7 : 1 Äthanol und 5,0 : 1 bis 7,2 : 1 Methylenchlorid zu Dihydroxy-3$\alpha$,7$\alpha$,5$\beta$-cholansäure verwendet wird.

6. Verfahren gemäss einem der Ansprüche 1 bis 5, dadurch gekennzeichnet, dass die Kristallisation in der Wärme durchgeführt wird.

7. Verfahren gemäss Anspruch 1 oder 2, dadurch gekennzeichnet, dass man die Dihydroxy-3$\alpha$,7$\alpha$,5$\beta$-cholansäure durch Trocknen des kristallisierten Produkts gewinnt.

8. Verfahren gemäss Anspruch 1 oder 2, dadurch gekennzeichnet, dass man die Dihydroxy-3$\alpha$,7$\alpha$,5$\beta$-cholansäure durch Trocknen des kristallisierten Produkts, anschliessende Auflösung des erhaltenen Produkts in einem Aceton-Wassergemisch, partielle Verdampfung dieses Gemisches und anschliessende Isolierung des erhaltenen kristallisierten Produkts gewinnt.

## Claims

1. Process for purifying dihydroxy-3$\alpha$, 7$\alpha$, (5$\beta$) cholanic acid, characterized in that the dihydroxy-3$\alpha$, 7$\alpha$, (5$\beta$) cholanic acid to be purified is crystallized from methylene chloride, the crystalline product formed is collected and from this is recovered the purified dihydroxy-3$\alpha$, 7$\alpha$, (5$\beta$) cholanic acid.

2. Process, according to claim 1, characterized in that the crystallisation is carried out in a crystallisation system with methylene chloride and an alcohol.

3. Process according to claim 1 or 2, characterized in that the crystallisation system is constituted by methylene chloride and an aliphatic alcohol.

4. Process according to claim 3, characterized in that the aliphatic alcohol is ethanol.

5. Process according to claim 4, characterized in that the crystallisation system is used in the ratios of 0.1 : 1 to 0.7 : 1 of ethanol and 5.9 : 1 to 7.2 : 1 of methylene chloride, with reference to dihydroxy-3$\alpha$, 7$\alpha$, (5$\beta$) cholanic acid.

6. Process according to any one of claims 1 to 5, characterized in that the crystallisation is carried out with heating.

7. Process according to claim 1 or 2, characterized in that the dihydroxy-3$\alpha$, 7$\alpha$, (5$\beta$) cholanic acid is recovered by drying the crystalline product.

8. Pocess according to claim 1 or 2, characterized in that the dihydroxy-3$\alpha$, 7$\alpha$, (5$\beta$) cholanic acid is recovered by drying the crystalline product, then dissolving the product obtained in an acetone/water mixture, partially evaporating the said mixture, then isolating the crystalline product obtained.